# EUROPEAN PATENT APPLICATION

(11) **EP 1 879 142 A2**
(43) Date of publication of application: **16.01.2008**
(21) Application number: 07252733.6
(22) Date of filing: 06.07.2007
(51) Int. Cl.: G06Q 50/00

(54) **Virtual human interaction system**

(30) Priority: 12.07.2006 GB 0613832
(71) Applicant: KEELE UNIVERSITY, Staffordshire ST5 5BG (GB)
(72) Inventor: Chapman, Stephen, Knutsford, Cheshire WA16 9QZ (GB); Bracegirdle, Luke, Stok-on-Trent, Staffordshire ST9 0JZ (GB)
(74) Representative: Vaughan, Christopher Tammo

(57) **Abstract**

A virtual human interaction system is described for use on a PC or web-enabled computer which facilitates the training and education of medical services practitioners such as doctors, nurses, pharmacists and the like by allowing them to virtually interact with a virtual patient delivered by the system and displayed on the computer screen. The system embodies a plurality of cases, and for each case, there are a number of possible outcomes, depending on the choices made by the medical services practitioner at each stage in a particular case. Such choices are made in the form of user input to the system through the computer, and each case consists of a decision tree element consisting of branch elements from which the decision tree may branch in one or more directions toward further branch elements or tree termini, the user input cause the system to move through the decision tree of the case. Each branch element and terminus includes descriptors of a particular condition of the virtual human at that time in the specific case, and these are displayed to the user at each specific stage in the case to provide the user with a current indication of the well being of the virtual patient. Together with the virtual patient displayed by the system, also incorporated into the system are a plurality of appearance descriptors which can be applied to the virtual patient by the system so as to cause a change in the appearance thereof, said descriptors being based on real-life human conditions which affect the physical appearance of humans generally and which are thus mimicked in the virtual patient. In accordance with the invention, the system causes the appearance of the virtual patient to change by applying one or more of said appearance descriptors to said virtual patient as the system moves through the decision tree in response to user input. The resulting effect is to provide users with an almost real-time indication of their actions on patients.

## Description

This invention relates to a virtual human interaction system, and more particularly to a virtual human interaction system capable of being provided over local or disparate computer networks to users at one or more terminals and whereat users are presented with a situation involving one or more humans, which are virtually represented on-screen at said terminal, and with which said users must interact by providing one or more inputs at the terminal.

More specifically, this invention relates to a virtual patient system ideally intended for trainee medical practitioners to help them to learn or enhance their diagnostic skills based on a simulated doctor/patient scenario which is virtually represented on screen. Of course, while the following description relates almost exclusively to the use of the invention in the medical industry for the purpose specified, the reader will instantly become aware that the invention has a potentially much wider application in the training and education fields generally, and therefore the invention should be considered as encompassing such applications.

### BACKGROUND

Virtual education and/or training systems which involve some type of background computer program coupled with images and/or video files (e.g. mpeg, avi and the like) for display on-screen are well established. Furthermore, such systems can be provided both locally, in terms of being provided and loaded on an individual, stand-alone, non-networked PC, and in distributed fashion, whereby the system is stored centrally and delivered either physically in terms of being downloadable to suitably networked PCs, or virtually in terms of the program being executable at the server side and the results of the execution (which is to some extent controlled by the user input at the networked PC) are then transmitted by HTML or other suitable format so that the display on the user's PC can be caused to change as program execution continues.

Indeed there are many examples of such systems. One example can be found at http://medicus.marshall.edu/ and is entitled "The Interactive Patient". In this system, which is presented over the internet in HTML format, the user clicks through a series of stages, such as "History", "Physical Exam", "X-Ray Exam", and "Diagnosis", and with each stage that is clicked, a web page is presented to a user on which some explanatory text concerning the condition, symptoms, and medical history of a virtual patient is provided, together with a static, real-life photo image of a doctor greeting, examining, interrogating or otherwise dealing with a patient. Of course, both doctor and patient may be represented by actors, and in the case of systems where video footage is provided to users, such actors would be previously instructed how to behave during filming according to the particular notional plight of the patient, e.g. the actor playing the patient is told to limp as a result of having a notional sprained ankle.

This system is typical of many available on the web, in that a student is presented with a patient case to read, optionally provided with some patient medical history or medical records, and is then presented with a number of related options. Such systems are fundamentally limited in that they can relate only to one possible situation. For example, the user will be presented with a case to which the photos or video footage used are exclusively appropriate. Additionally, the text used in describing the case is most likely to be hard-coded into the website being provided, with the result that a total re-design is required if such systems are to be useful in training users in other situations. Indeed, in the case of the training of medical practitioners, it is almost imperative that they be exposed to as many different cases and patient diagnosis scenarios as is possible to provide them with as well rounded and comprehensive a training as is possible. In this regard, the type of system immediately previously described is wholly inadequate.

Other systems of this type can found at:
http://courses.pharmacy.unc.edu/asthma/
http-//medguides.medicines.org.uk/ai/ai.aspx?id=AI1005&name=Becotide
http://research.bidmc.harvard.edu/VPTutorials/
http://radiography.derby.ac.uk/NOS Conference/Dawn%20Skelton%202.pd

As an advance on the above, it has been proposed to use virtual reality to enhance the training/user experience. A technical paper entitled "Virtual Patient: a Photo-real Virtual Human for VR-based Therapy" by Bernadette KISS, Balázs BENEDEK, Gábor SZIJÁRTÓ, Gábor CSUKLY, Lajos SIMON discussed a high fidelity Virtual Human Interface (VHI) system which was developed using low-cost and portable computers. The system features realtime photo-realistic digital replicas of multiple individuals capable of talking, acting and showing emotions and over 60 different facial expressions. These "virtual patients" appear in a high-performance virtual reality environment featuring full panoramic backgrounds, animated 3D objects, behaviour and A.I. models, a complete vision system for supporting interaction and advanced animation interfaces. The VHI takes advantage of the latest advances in computer graphics. As such, it allows medical researchers and practitioners to create real-time responsive virtual humans for their experiments using computer systems priced under $2000.

In this document, the creation of Computer generated, animated humans in real-time is used to address the needs of emerging virtual-reality based medical applications, such as CyberTherapy, virtual patients, and digital plastic surgery. The authors developed an open architecture, low-cost and portable virtual reality system, called the Virtual Human Interface, which employs high resolution, photo-real virtual humans animated in real-time to interact with patients. It is said that this system offers a unique platform for a broad range of clinical and research applications. Examples include virtual patients for training and interviewing, highly realistic 3D environments for cue exposure therapy, and digital faces as a means to diagnose and treat psychological disorders. Its open architecture and multiple layers of interaction possibilities make it ideal for creating controlled, repeatable and standardized medical VR solutions. By virtue of the system proposed, the virtual patients can talk, act and express a wide range of facial expressions, emotions, and body gestures. Their motions and actions can be imported from MPEG4 or motion capture files or animated. An additional scripting layer allows researchers to use their own scripting controls implemented in XML, HTML, LUA, TCL/TK or TCP/IP.

They state that the system also runs in a browser environment over the Internet and supports multiple ways, such a live video feed, remote teleconferencing and even a virtual studio module (chroma-key) for the therapist to enter the patient's virtual space whether locally or remotely.

Despite the obvious advantages of providing a virtual patient as described above, and in particular the utility of such a system in bringing virtual doctor/patient encounters to life on screen, there are still drawbacks in that the designer of particular cases must ree-design each and every case, and adapt the virtual reality patient element accordingly for each case. As will immediately be appreciated, this represents a massive overhead, and a probably unworkable solution to the problem of providing trainees with a great variety of cases to study.

It has also been proposed to provide decision-based systems, such as can be found at:
http://www.hud.ac.uk/schools/hhs/departments/nursing/penfield site/default.htm

This system, developed by the University of Huddersfield, UK, creates a "virtual hospital" in HTML and other code, and is a computer-based learning tool for health care professionals that simulates the care context for patients within the environmental context of a General Hospital.

The system has been built from components that reflect typical aspects of a care environment e.g. patients, patient assessment forms, observations records etc. The components provide the facilitator with the means to support complex and imaginative patient based scenarios to satisfy key learning outcomes. The system is innovative in design and delivery of course materials as it encourages students to engage with nursing matter through vignettes and patient based scenarios within the context of application to patient care; and allows students to explore wider issues relating to management of the care environment through duty roster and resource management and exploration of evidence based practice.

In this system however, cartoon-like animations are provided on-screen as opposed to virtual full-size patients which can be displayed, and although the system provides a good overall "feel", it is deficient in that again it allows little scope for extensive development, for example by being scalable to include vast numbers of doctor/patient cases or prognosis/diagnosis scenarios.

A further disadvantage of all these systems is that none provide a medium whereby an educator can design his own cases for teaching purposes without some computer programming experience.

It is a primary object of the present invention to provide a system which overcomes the disadvantages of the prior art and provides a means whereby scenario analysis, diagnosis, and or prognosis can be combined with virtual reality technology for representing humans on-screen to provide a remarkable provide a remarkable learning experience.

### BRIEF SUMMARY OF THE DISCLOSURE

According to the invention, there is provided a virtual human interaction system for use on a user terminal, said system being adapted for a plurality of cases to which a number of possible outcomes can be achieved depending on the user input to the system at various stages through its delivery, each case consisting of at least a decision tree element consisting of branch elements from which the decision tree may branch in one or more directions toward further branch elements or tree termini, each branch element and terminus including descriptors of a particular condition of the virtual human at that time in the specific case,
said system comprising at least
- a virtual human representation element capable of being displayed graphically to appear as a virtual human on screen, and
- a plurality of appearance descriptor elements capable of interacting with the virtual human representation element so as to cause a change in the appearance thereof, said appearance descriptor elements being based on real-life human conditions which affect the physical appearance of humans generally and which are thus mimicked in the virtual human
characterised in that
the system causes the appearance of the virtual human to change by applying one or more of said appearance descriptor elements to said virtual human representation element when the system is caused to be at one the branch elements or termini as a result of user input at said terminal.

Further features of the invention are provided in the dependent claims appended hereto.

The fundamental advantage of this invention is its ability to starkly identify, through the appearance of the on-screen virtual patient, exactly what the effect on such a patient would have been in real-life had the user acted in the way he did during the virtual case study provided by the system. For example, if case offered the option to the user of prescribing various drugs to treat the virtual patient's condition, and the user chose the wrong drug, the system could, almost in real-time, display the (possibly fatal) effects of incorrect prescription. For instance, a set of descriptors (possibly code fragments, mini-applications, or other graphics tools) could be applied to the virtual patient to cause the displayed figure to faint, vomit, turn different shades of colours, become blotchy, sweat, become feverish, to collapse, and possibly ultimately, to die. Of course, many other conditions can be defined and described in suitable code, tools, applications or other format compatible with the system.

It is worth mentioning at this time that the effects of the system on test candidates is so startling that most remember the experience very clearly. When compared to studying comparatively dry and dull textbooks, the system provides a marked improvement. One of the reasons behind this improvement is that the patient condition descriptors (i.e. "the faint", "the vomit", "the collapse", "the death") is independent of the particular virtual patient representation. Accordingly, any virtual reality figure can be incorporated into the system, and it is to this basic figure that the various conditions can be applied. Not only does this make the system very flexible (for instance, it is thus very simple to change the virtual representation from a man to a woman), but also it provides the system as a whole with advanced realism. For example, it could easily be possible to virtually represent someone the user knew in real life, which would further enhance the experience of using the system.

A specific description of the invention will now be provided with reference to the accompanying drawings

### BRIEF DESCRIPTION OF THE DRAWINGS

Figure 1 provides a diagrammatic representation of the system as a whole, and
Figure 2 shows a possible decision tree structure suitable for a case involving a patient who is an asthma sufferer.

### DETAILED DESCRIPTION

As a first part of this description, the method by which cases are designed for the system is described.

The first step in designing a case is Patient Selection. In designing a new case for use with the system according to the invention, it needs to focus on a single patient.
Different patients can be used for individual cases, and information on other people (e.g. family members) can be provided in the branch element/terminus descriptors if relevant.

During this phase of development, it is necessary to describe the patient's profile. The system requires information about the patient such as their description (gender, age, height, weight etc.), previous medical history and any social history. It is perceived by the applicant herefor that after a number of cases have been designed, the system may be extended to develop a 'patient population' - a small set of patients that can be perceived as members of a virtual community. Such a resource would allow case designers to select a patient from the patient population or examine the effect of their decision across the entire virtual patient population.

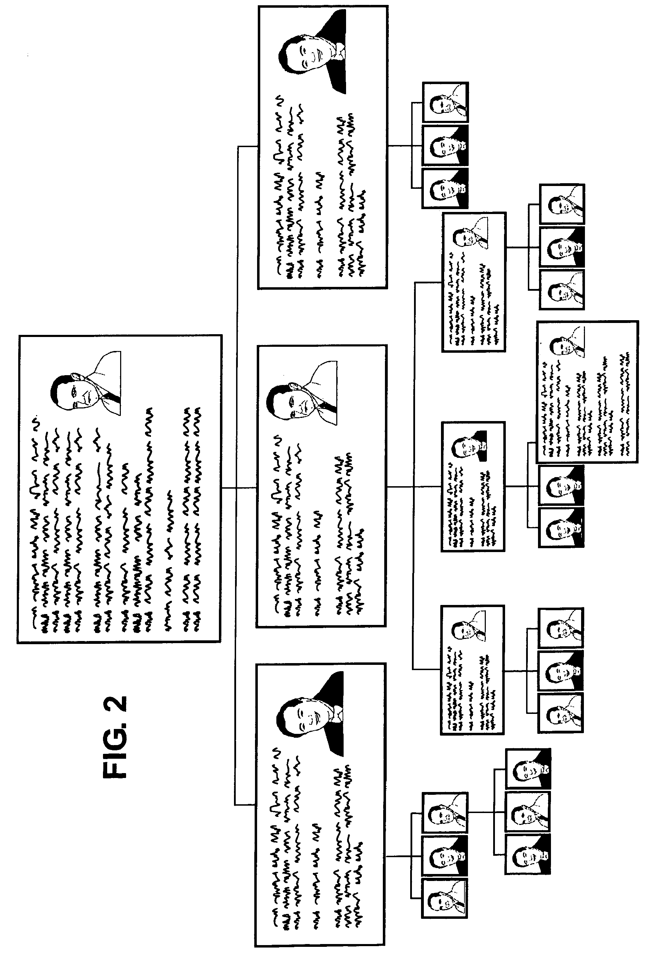

A particular case is created by designing a number of scenarios that are linked by the decisions that can be taken. This relates to the decision tree aspect of the invention, which may most usefully be mapped out in a flowchart or organisational chart, such as that shown in Figure 2. Each of the boxes can be thought of as branch elements (i.e. elements from which a branch extends or is possible) or termini (i.e. from which no further branch is possible). In each box, there is provided some text indicative of the patients physical state at that stage in the diagnosis procedure. Also provided in each branch element are a series of options or other means by which a user can enter information or make a selection. This user input is then fed back to the system to allow it to determine, according to the decision tree, which branch element to display next.

As can be seen in the Figure, a case will often have more than one final scenario, depending on the various options which are offered to and chosen by the user.

A case is made up of many scenarios which need to be described individually to support the decisions that can be taken. To begin writing a case, it is necessary to consider the following pieces of information for each scenario:
Audience
The type of student for whom the case is designed (e.g. Pharmacy, Medical, Nursing students). As a case has many scenarios, the audience does not always have to be the same for each scenario. For example by changing the audience, it is possible to design a case to allow a group of students from various health disciplines to work together on a single case.

### Description

Each scenario must fully described as it will appear to the student (e.g. Anne walks into the pharmacy complaining of...).

### Additional Information

The system can easily be adapted to provide additional information in the form of attachments to the user, so word documents, http links, specific pictures and the like can be included, and the system can the student to refer to such to support their decision for each scenario.

### Decisions

Unless an outcome scenario is being described for a case, it is necessary to provide two or more decisions for each scenario described, together with branch information, i.e. where each decision should lead. A simple numbering scheme for the scenarios would allow one scenario to reference another. In this manner, it is of course possible to reuse scenarios, so that a number of decisions can result in the display of the same scenario. In the system, it may also be necessary to categorise each decision into three types. All decisions may be broadly categorised as (a) treating the patient, (b) referring the patient to another healthcare professional or (c) giving advice to the patient.

### Multimedia

With each scenario, it is possible (although not mandatory) to request visualisation of one or more key points of the scenario through virtual patient technologies incorporated into the system.

An example of a case description is provided below.

### Patient Description

Retired Teacher, Caucasian, Weight = 88kg
Married, husband a heavy smoker
Two Children (Luke and Jessica), now 31 and 29

Anne has suffered with asthma since childhood, suffering 3 exacerbations in the past 12 months. She had a total hysterectomy at age 48, menorrhagia & prolapse FH of CVD. Her mother died following a stroke at age 76, prior to this she had a succession of TIAs and had moved in with Anne and her husband. Husband worked in management for the local coal board and was retired on grounds of ill health (arthritis) in 1996 (age 62).

Anne buys analgesics regularly from the local pharmacy for her husband (Co-Codamol 8/500) as he doesn't like to bother the GP for such 'minor' medications.
Anne doesn't have any help at home, she does her own cooking and cleaning and when her asthma is ok her mobility and exercise tolerance is good. She was advised to increase her activity levels a few years ago and has started to walk the dog more since her husband is becoming increasingly unable to walk long distances without significant pain.

### Scenario Number: 1

Audience: Pharmacy Students & Medical Students Only

### Description

Anne has been asthmatic for many years. She has attended her review annually at the surgery and her prescription has stayed pretty constant for some time. There have been a number of acute exacerbations of her asthma in the past 12 months and she attends for review at the surgery following an Accident and Emergency admission 5 days previously...

### Additional Information

[include word documents, links etc. to additional resources for student consideration]

### Decisions

Category: (a) treating the patient
   increase Beclometasone to 250mcg 2 puffs bd MDI (goes to scenario 3)
Category: (c) giving advice to the patient
   check inhaler technique (goes to scenario 4)
Category: (a) treating the patient
   switch to Easi-breathe devices (goes to outcome scenario 2)

### Multimedia

Picture of patient attending her annual review and taking a peak flow measurement. [the description of the remaining 9 scenarios in this case is precluded here in the interest of brevity, but the format is generally similar to the above].

In order to the present the pre-designed cases in an informative, useful and striking manner, the system is designed as follows.

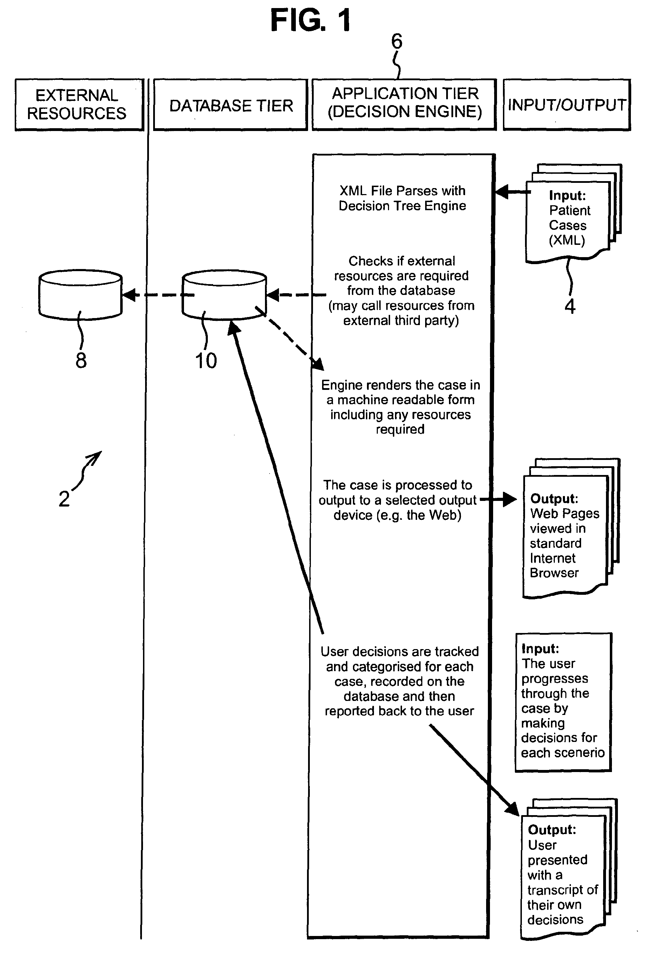

Referring to Figure 1, the system 2 comprises of three main components to deliver the core functionality. These are referred to as:
(1) The Patient Case File 4 - This is an XML based file that drives the content in each case. The file format can be generated with supporting applications to allow case designers with little, or no technical knowledge to create new cases for use with the system.
   This file uses a XML definition to allow the decision engine to parse the file and process its contents. The files employ a decision tree to traverse the various scenarios a patient case may have, depending on the decisions taken within a case.
(2) The Decision Engine 6 - This is responsible for parsing the Patient Case File and rendering the content into a machine readable format. The decision engine 6 is also responsible for calling external resources 8 that the case may need to render the case (e.g. documents, images, animations/sound files) and then formats the case back to the user via a standard output format (e.g. web page).
   In accordance with the invention, the external resources also includes the descriptors which can be applied to the virtual human, the computer-readable representation of which is similarly retained in the database.
   The engine also tracks the decisions taken by a user in each case and then passes this data onto a database 10 for recording. This information is then used when a user wishes to examine a transcript of what decisions the user made for a specific case.
(3) The Database 10 - The database is responsible for tracking decisions taken within each case and to keep a record of the location of external resources that may be required to render a case (e.g. animation files).

The database is also referred to when a user wishes to recall their decisions within a case. This information is also used at a higher level, so that cases designers can examine what type of decisions are being made in their case and if additional supporting information needs to be supplied to the user to improve the decision making process.

At a technical level, to allow the decision engine to parse the XML file so that the system can provide this functionality, information is declared in the XML file as a series of special XML tags.

At the start of the file, a tag is declared identifying the patient the case applies to:
<patient id=01>Anne Phillips...

Each scenario is then declared via series of scenario tags that describe what is happening to the patient at this stage of the case. Typically, you would expect to see a series of scenario tags to make up the various scenarios of each case.
<scenario01>Anne complains of breathlessness.....what do you do?
</scenario01 >

Within each scenario, additional information can be provided to the user (via hyperlinks) before they make their decision. This is declared in the file as follows:
<scenario011ink url="CMP.doc">
CMP
</scenario01link>
<scenario01link url="http://www.sign.ac.uk">
SIGN/BTS Guidelines
</scenario01ink>
<scenario01link url="http://www.nice.org.uk">
NICE
</scenario01link>

Decisions are then declared, being those decision applicable to the particular scenario. Each of these decisions are categorised via the "Type" attribute and is recorded back to the database accordingly.
<scenario01option type="a">increase Beclometasone to 250mcg...
</scenario01 option>
<scenario01option type="b">check inhaler technique
</scenario01 option>
<scenario01 option type="c">switch to easi-breathe devices
</scenario01 option>

As a next part of the file, the decisions are mapped to paths within the decision tree to allow the case to traverse the tree correctly. Each scenario is made anonymous by its id value and referenced in the XML file thus:
<scenario01 path>02</scenario01 path>
<scenario01 path>03</scenario01 path>
<scenario01 path>04</scenario01 path>

A tag is also included in the XML file which calls an external multimedia resource, and in particular an emotional or physical descriptor file which can be applied to a default virtual human (e.g. avatar) in memory, in accordance with the invention. This may be an image file, sound file or an animation to cause the avatar to respond in a predefined way. This may involve using a file from an external media suppler and can be declared in the XML file as follows:
<scenario01 resource file="patientol /emotion s/pain.flv">02
</scenario01 resource>

Such animation files need to be designed before the XML file can reference them. However animations are designed and can be invoked at a code level and applied to different patients. Therefore it is possible for the invention to call on a database of animations (using a combination of external and in-house developed multimedia resources) to invoke an emotion in the patient across a number of cases.

Thus, for common actions (e.g. smiling, angry, sad), these can be designed for all patients in one process and therefore allows for an extensive population of animations which the XML file can reference via this tag.

It is important to note that supporting software applications may be produced which guide a designer through writing a case. This software will automatically generate the XML required in a Patient Case File.

In summary therefore, a virtual human interaction system is described for use on a PC or web enabled computer, which facilitates the training and education of medical services practitioners such as doctors, nurses, pharmacists and the like by allowing them to virtually interact with a virtual patient delivered by the system and displayed on the computer screen. The system embodies a plurality of cases, and for each case, there are a number of possible outcomes, depending on the choices made by the medical services practitioner at each stage in a particular case. Such choices are made in the form of user input to the system through the computer interface, and each case consists of a decision tree element consisting of branch elements from which the decision tree may branch in one or more directions toward further branch elements or tree termini, the user input cause the system to move through the decision tree of the case. Each branch element and terminus includes descriptors of a particular condition of the virtual human at that time in the specific case, and these are displayed to the user at each specific stage in the case to provide the user with a current indication of the well being of the virtual patient. Together with the virtual patient displayed by the system, also incorporated into the system are a plurality of appearance descriptors which can be applied to the virtual patient by the system so as to cause a change in the appearance thereof, said descriptors being based on real-life human conditions which affect the physical appearance of humans generally and which are thus mimicked in the virtual patient. In accordance with the invention, the system causes the appearance of the virtual patient to change by applying one or more of said appearance descriptors to said virtual patient as the system moves through the decision tree in response to user input. The resulting effect is to provide users with an almost real-time indication of their actions on patients.

## Claims

1. A virtual human interaction system for use on a user terminal, said system being adapted for a plurality of cases to which a number of possible outcomes can be achieved depending on the user input to the system at various stages through its delivery, each case consisting of at least a decision tree element consisting of branch elements from which the decision tree may branch in one or more directions toward further branch elements or tree termini, each branch element and terminus including descriptors of a particular condition of the virtual human at that time in the specific case, said system comprising at least
- a virtual human representation element capable of being displayed graphically to appear as a virtual human on screen, and
- a plurality of appearance descriptor elements capable of interacting with the virtual human representation element so as to cause a change in the appearance thereof, said appearance descriptor elements being based on real-life human conditions which affect the physical appearance of humans generally and which are thus mimicked in the virtual human
**characterised in that**
the system causes the appearance of the virtual human to change by applying one or more of said appearance descriptor elements to said virtual human representation element when the system is caused to be at one the branch elements or termini of the decision tree as a result of user input at said terminal.

2. A system according to claim 1 wherein the system causes the appearance of the virtual human to change immediately with the change in position of the system within the decision tree, thus giving a realistic indication to the user of the effects of their inputs to the system.

3. A system according to any preceding claim wherein the cases to which the system is adapted are virtual ones in which a medical services provider interacts with a virtual patient displayed on-screen.

4. A system according to claim 3 wherein
the virtual patient suffers from a predetermined ailment or condition initially unknown to the medical services provider,
the branch element or termini descriptors, which may be complete or incomplete as far the condition of the virtual patient is concerned, are provided successively by the system to the medical services practitioner as information which should be indicative of, or at least suggestive of the particular ailment or condition, and
the system provides one or more options to the medical services practitioner from which a selection of one or more options is made, said options being returned to the system which thus cause the system to move to the next branch element or terminus in the decision tree, display the next descriptor associated therewith, and optionally to cause the appearance of the virtual patient to change if so demanded by the case and the previous user input.
